(19) Europäisches Patentamt
European Patent Office
Office européen des brevets

(11) EP 1 100 433 B1

(12) EUROPÄISCHE PATENTSCHRIFT

(45) Veröffentlichungstag und Bekanntmachung des Hinweises auf die Patenterteilung:
**16.06.2004 Patentblatt 2004/25**

(21) Anmeldenummer: **99939395.2**

(22) Anmeldetag: **22.07.1999**

(51) Int Cl.[7]: **A61K 7/00**, A61K 7/48, A61K 7/42

(86) Internationale Anmeldenummer:
**PCT/EP1999/005243**

(87) Internationale Veröffentlichungsnummer:
**WO 2000/007549 (17.02.2000 Gazette 2000/07)**

# (54) EMULGATORFREIE UND BORNITRID ENTHALTENDE FEINDISPERSE SYSTEME VOM TYP ÖL-IN-WASSER UND WASSER-IN-ÖL

FINELY DISPERSED EMULSIFIER-FREE SYSTEMS OF THE OIL-IN-WATER AND WATER-IN-OIL TYPE, CONTAINING BORON NITRIDE

SYSTEMES FINEMENT DISPERSES SANS EMULSIFIANT, CONTENANT DU NITRURE DE BORE, DU TYPE HUILE DANS EAU ET EAU DANS HUILE

(84) Benannte Vertragsstaaten:
**AT BE CH CY DE DK ES FI FR GB GR IE IT LI LU MC NL PT SE**

(30) Priorität: **01.08.1998 DE 19834820**

(43) Veröffentlichungstag der Anmeldung:
**23.05.2001 Patentblatt 2001/21**

(73) Patentinhaber: **Beiersdorf Aktiengesellschaft 20245 Hamburg (DE)**

(72) Erfinder:
- **GERS-BARLAG, Heinrich D-25495 Kummerfeld (DE)**
- **MÜLLER, Anja D-23843 Rümpel (DE)**

(56) Entgegenhaltungen:
**DE-A- 4 303 983** **FR-A- 2 746 301**

- **INTROINI, C.: "Nitruro di Boro. Nuova Materia Prima" COSMET. TOILETRIES, ED. ITAL., Bd. 18, Nr. 3, 1997, Seiten 26-27,30-32, XP000853905 & CHEMICAL ABSTRACTS, vol. 127, no. 17, 27. Oktober 1997 (1997-10-27) Columbus, Ohio, US; abstract no. 238872, INTROINI, C.: "Boron nitride. A new raw material. Comparison with cosmetic powders."**
- **INTRONIN, C.: "Nitruro di Boro. Nuevo ingrediente nei prodotti per il corpo e nei solari" COSMET. TOILETRIES, ED. ITAL., Bd. 18, Nr. 2, 1997, Seiten 71,73,75-76, XP000853904 & CHEMICAL ABSTRACTS, vol. 127, no. 10, 8. September 1997 (1997-09-08) Columbus, Ohio, US; abstract no. 140174,**

EP 1 100 433 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft emulgatorfreie feindisperse Systeme vom Typ Öl-in-Wasser und Wasser-in-Öl, bevorzugt als kosmetische oder dermatologische Zubereitungen.

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Um die dauerhafte Dispergierung einer Flüssigkeit in einer anderen zu erreichen, ist bei Emulsionen im herkömmlichen Sinn der Zusatz einer grenzflächenaktiven Substanz (Emulgator) notwendig. Emulgatoren weisen einen amphiphilen Molekülaufbau auf, bestehend aus einem polaren (hydrophilen) und einem unpolaren (lipophilen) Molekülteil, die räumlich voneinander getrennt sind. In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Emulgatoren setzen die Grenzflächenspannung zwischen den Phasen herab, indem sie an der Grenzfläche zwischen beiden Flüssigkeiten angeordnet sind. Sie bilden an der Phasengrenze Öl/Wasser Grenzflächenfilme aus, wodurch dem irreversiblen Zusammenfließen der Tröpfchen entgegengewirkt wird. Zur Stabilisierung von Emulsionen werden häufig Emulgatorgemische verwendet.

Herkömmliche Emulgatoren können entsprechend ihrem hydrophilen Molekülteil in ionische (anionische, kationische und amphotere) und nichtionische untergliedert werden:

- Das wohl bekannteste Beispiel eines anionischen Emulgators ist die Seife, als die man gewöhnlich die wasserlöslichen Natrium- oder Kaliumsalze der gesättigten und ungesättigten höheren-Fettsäuren bezeichnet.
- Wichtige Vertreter der kationischen Emulgatoren sind die quartären Ammonium-Verbindungen.
- Der hydrophile Molekülteil nichtionischer Emulgatoren besteht häufig aus Glycerin, Polyglycerin, Sorbitanen, Kohlenhydraten bzw. Polyoxyethylenglykolen und ist meistens über Ester- und Etherbindungen mit dem lipophilen Molekülteil verknüpft. Dieser besteht üblicherweise aus Fettalkoholen, Fettsäuren oder Isofettsäuren.

Durch Variation der Struktur und der Größe des polaren und des unpolaren Molekülteils lassen sich Lipophilie und Hydrophilie von Emulgatoren in weiten Grenzen verändern.

Entscheidend für die Stabilität einer Emulsion ist die richtige Auswahl der Emulgatoren. Dabei sind die Charakteristiken aller im System enthaltenen Stoffe zu berücksichtigen. Betrachtet man z. B. Hautpflegeemulsionen, so führen polare Ölkomponenten und beispielsweise UV-Filter zu Instabilitäten. Neben den Emulgatoren werden daher noch andere Stabilisatoren verwendet, die beispielsweise die Viskosität der Emulsion erhöhen und/oder als Schutzkolloid wirken.

Emulsionen stellen einen wichtigen Produkttyp im Bereich kosmetischer und/oder dermatologischer Zubereitungen dar.

Kosmetische Zubereitungen werden im wesentlichen zur Hautpflege benutzt. Hautpflege im kosmetischen Sinn ist in erster Linie, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird. Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Kosmetische Zubereitungen werden auch als Desodorantien verwendet. Solche Formulierungen dienen dazu, Körpergeruch zu beseitigen, der entsteht, wenn der an sich geruchlose frische Schweiß durch Mikroorganismen zersetzt wird.

Medizinische topische Zusammensetzungen enthalten in der Regel ein oder mehrere Medikamente in wirksamer Konzentration. Der Einfachheit halber wird zur eindeutigen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

An sich ist die Verwendung der üblichen Emulgatoren in kosmetischen oder dermatologischen Zubereitungen unbedenklich. Dennoch können Emulgatoren, wie letztlich jede chemische Substanz, im Einzelfalle allergische oder

auf Überempfindlichkeit des Anwenders beruhende Reaktionen hervorrufen.

So ist beispielsweise bekannt, daß bestimmte Lichtdermatosen durch gewisse Emulgatoren, aber auch durch verschiedene Fette und gleichzeitige Exposition von Sonnenlicht ausgelöst werden. Solche Lichtdermatosen werden auch "Mallorca-Akne" genannt. Es hat daher nicht an Versuchen gefehlt, die Menge an üblichen Emulgatoren auf ein Minimum, im Idealfall sogar vollständig zu reduzieren.

Eine Reduktion der benötigten Emulgatormenge kann z. B. erreicht werden, wenn ausgenutzt wird, daß feinstverteilte Feststoffteilchen eine zusätzlich stabilisierende Wirkung haben. Dabei kommt es zu einer Anreicherung des festen Stoffes an der Phasengrenze Öl/Wasser in Form einer Schicht, wodurch Zusammenfließen der dispersen Phasen verhindert wird. Von wesentlicher Bedeutung sind dabei nicht die chemischen, sondern die Oberflächeneigenschaften der Feststoffpartikel.

Pickering stellte um 1910 Paraffin-Wasser-Emulsionen her, die nur durch den Zusatz verschiedener Feststoffe wie basisches Kupfersulfat, basisches Eisensulfat oder andere Metallsulfate stabilisiert wurden. Diese Art von Emulsionen wird daher auch als Pickering-Emulsion bezeichnet. Für diese Form von Emulsionen postulierte Pickering die folgenden Bedingungen:

(1) Die Feststoffpartikel sind nur dann zur Stabilisierung geeignet, wenn sie deutlich kleiner sind als die Tröpfchen der inneren Phase und nicht zur Agglomeratbildung neigen.

(2) Wichtige Eigenschaft eines emulsionsstabilisierenden Feststoffs ist außerdem seine Benetzbarkeit. D.h. zur Stabilisierung einer O/W-Emulsion muß der Feststoff beispielsweise leichter von Wasser als von Öl benetzbar sein.

Sozusagen die Urformen von Pickering-Emulsionen tauchten zunächst als unerwünschte Nebeneffekte bei unterschiedlichen technischen Prozessen auf, wie z. B. bei der sekundären Erdölförderung, der Extraktion von Bitumen aus Teersand und anderen Trennungsverfahren, an denen zwei nicht miteinander mischbare Flüssigkeiten und feine, dispergierte Feststoffpartikel beteiligt sind. Im allgemeinen handelt es sich hierbei um W/O-Emulsionen, die durch mineralische Feststoffe stabilisiert werden. Dementsprechend stand die Untersuchung entsprechender Systeme, wie z. B. der Systeme Öl-Wasser-Ruß oder Öl-Wasser-Schieferstaub zunächst im Mittelpunkt der Forschungsaktivitäten.

Grundlegende Untersuchungen haben dabei gezeigt, daß ein Charakteristikum für eine Pickering-Emulsion ist, daß die Feststoffpartikel an der Grenzfläche zwischen den beiden flüssigen Phasen angeordnet sind und dort sozusagen eine mechanische Barriere gegen die Vereinigung der Flüssigkeitströpfen bilden.

Eine relativ neue technische Entwicklung ist es, Pickering-Emulsionen als Grundlage für kosmetische oder dermatologische Zubereitungen zu verwenden.

Eine Möglichkeit, eine Feststoffstabilisierung in einer kosmetischen oder dermatologischen Zubereitung vorzunehmen, ist nach May-Alert *(Pharmazie in unserer Zeit, 15. Jahrg. 1986, Nr. 1,* 1-7) beispielsweise, Emulgatorgemische zu verwenden, die sowohl anionische als auch kationische Tenside enthalten. Da beim Zusammengeben von Anion- und Kationtensiden immer unlösliche, elektroneutrale Verbindungen ausfallen, läßt sich durch gezieltes Ausfällen dieser neutralen Tenside in der Grenzfläche Öl/Wasser eine zusätzliche Feststoffstabilisierung im Sinne einer Pickering-Emulsion erreichen.

Die Europäische Offenlegungsschrift 0 686 391 beschreibt Emulsionen vom Typ Wasser-in-Öl, die frei von oberflächenaktiven Substanzen sind und nur durch Feststoffe stabilisiert werden. Zur Stabilisierung werden hier sphärische Polyalkylsilsesquioxan-Partikel eingesetzt, die einen Durchmesser von 100 nm bis zu 20 μm haben. Diese Emulsionen können nach dem oben gesagten als Pickering-Emulsionen bezeichnet werden.

Der Stand der Technik kennt neben den beschriebenen Pickering-Emulsionen weitere emulgatorfreie, feindisperse kosmetische oder dermatologische Zubereitungen, die im allgemeinen als Hydrodispersionen bezeichnet werden. Hydrodispersionen stellen Dispersionen einer flüssigen, halbfesten oder festen inneren (diskontinuierlichen) Lipidphase in einer äußeren wäßrigen (kontinuierlichen) Phase dar.

Bei Hydrodispersionen einer flüssigen Lipidphase in einer äußeren wäßrigen Phase kann die Stabilität beispielsweise dadurch gewährleistet werden, daß in der wäßrigen Phase ein Gelgerüst aufgebaut wird, in welchem die Lipidtröpfchen stabil suspendiert sind. Die Deutsche Offenlegungsschrift 44 25 268 beschreibt stabile feindisperse, emulgatorfreie kosmetische oder dermatologische Zubereitungen vom Typ Öl-in-Wasser, die neben einer Öl- und einer Wasserphase einen oder mehrere Verdicker aus der Gruppe der Acrylsäurepolymere, Polysaccharide und deren Alkylether enthalten; wobei für diese Verdicker eine Grenzflächenspannungsemiedrigung nicht meßbar sein darf.

Basierend auf ähnlichen Hydrodispersionen werden in der Deutschen Offenlegungsschrift 43 03 983 kosmetische oder dermatologische Lichtschutzformulierungen offenbart, die im wesentlichen frei von Emulgatoren sind, wobei in die Lipidphase der Hydrodispersion anorganische Mikropigmente eingearbeitet sind, die als UV-Filtersubstanzen dienen.

INTROINI, C.: 'Nitruro di Boro. Nuova Materia Prima' COSMET. TOILETRIES, ED. ITAL, Bd. 18, Nr. 3, 1997, Seiten 26-27,30-32, XP000853905 offenbart den Vergleich zwischen Bomitrid und $TiO_2$ bezüglich der UV-filterfähigkeit, der Weißwerte und der Adhäsion. Im Ergebnis wird festgestellt, daß Bornitrid Eigenschaften besitzt, die es für die

Verwendung in kosmetischen Zubereitungen geeignet erscheinen läßt.

Aufgabe der vorliegenden Erfindung war, den Stand der Technik um kosmetische oder dermatologische Zubereitungen zu bereichern, in welchen auf jeglichen Einsatz von Emulgatoren herkömmlicher Art verzichtet werden kann.

Erstaunlicherweise wird diese Aufgabe gelöst durch
kosmetische oder dermatologische Zubereitungen, die feindisperse Systeme vom Typ Öl-in-Wasser oder Wasser-in-Öl darstellen, enthaltend

1. eine Ölphase,
2. eine Wasserphase,
3. Bornitrid, welches sowohl hydrophile als auch lipophile Eigenschaften zeigt, welches also amphiphilen Charakter besitzt und sich an der Grenzfläche Wasser/Öl anordnet und
4. höchstens 0,5 Gew. % eines oder mehrerer Emulgatoren

sowie
gegebenenfalls weitere kosmetische oder pharmazeutische Hilfs-, Zusatz- und/oder Wirkstoffe enthaltend.

Es ist erfindungsgemäß besonders vorteilhaft, wenn die Zubereitungen deutlich weniger als 0,5 Gew.-% eines oder mehrerer Emulgatoren enthalten bzw. sogar gänzlich emulgatorfrei sind.

Die erfindungsgemäßen Zubereitungen stellen Abmischungen aus Ölen bzw. öllöslichen Substanzen und Wasser bzw. wasserlöslichen Komponenten dar, die durch den Zusatz der Bomitridpartikel stabilisiert werden und keinen Emulgator im herkömmlichen Sinn enthalten müssen. Die Stabilisierung wird dadurch erreicht, daß sich die Bomitridpartikel an die Tröpfchen der dispersen Phase anlagem und sozusagen eine mechanische Barriere bilden, die das Koaleszieren, d. h. das Zusammenfließen der Tröpfchen verhindert.

Die erfindungsgemäßen Zubereitungen sind in jeglicher Hinsicht überaus befriedigende Präparate, die erstaunlicherweise höchst variabel in ihrem Wasser-Fettphasen-Verhältnis sind und zudem gegenüber dem Stand der Technik den Vorteil bieten, daß hohe Mengen an Ölen in Wasser stabil eingearbeitet werden können. Es war zudem überraschend, daß bei Befolgung der hiermit offenbarten Lehre zum technischen Handeln sowohl Pickering-Emulsionen vom Typ Wasser-in-Öl als auch Pickering-Emulsionen vom Typ Öl-in-Wasser hergestellt werden können.

Vorteilhaft im Sinne der vorliegenden Erfindung sind beispielsweise die im folgenden aufgelisteten Bomitride:

| Handelsname | erhältlich bei |
|---|---|
| Boron Nitride Powder | Advanced Ceramics |
| Boron Nitride Powder | Sintec Keramik |
| Ceram Blanche | Kawasaki |
| HCST Boron Nitride | Stark |
| Très BN® | Carborundum |
| Wacker-Bornitrid BNP | Wacker-Chemie |

Vorteilhaft ist es, den mittleren Partikeldurchmesser der verwendeten Bomitridpartikel kleiner als 20 µm, besonders vorteilhaft kleiner als 15 µm zu wählen.

Pickering-Emulsionen im Sinne der vorliegenden Erfindung werden ebenfalls vorteilhaft durch Bomitridpartikel stabilisiert, die oberflächlich wasserabweisend behandelt ("gecoatet") sind, wobei gleichzeitig der amphiphile Charakter gebildet werden bzw. erhalten bleiben soll.

Eine vorteilhafte Beschichtung der Bomitridpartikel besteht aus Dimethylpolysiloxan (auch: Dimethicone), einem Gemisch vollmethylierter, linearer Siloxanpolymere, die endständig mit Trimethylsiloxy-Einheiten blockiert sind. Vorteilhaft sind beispielsweise die bei der Firma Carborundum unter der Handelsbezeichnung Tres BN® UHP 1106 erhältlichen, mit Dimethicone behandelten Bomitridpartikel.

Vorteilhaft ist ferner eine Beschichtung der Bomitridpartikel mit Polymethylhydrogensiloxan, einem linearen Polysiloxan, welches auch als Methicone bezeichnet wird. Vorteilhafte, mit Methicone behandelte Bornitridpartikel sind beispielsweise die bei der Firma Carborundum unter der Handelsbezeichnung Très BN® UHP 1107 erhältlichen.

Es ist ferner vorteilhaft, wenngleich nicht zwingend, die erfindungsgemäßen amphiphilen Bornitridpartikel mit weiteren amphiphilen Pigmenten zu kombinieren, welche gegebenenfalls auch zur Stabilisierung der Pickering-Emulsionen beitragen können.

Solche Pigmente sind beispielsweise mikronisierte, anorganische Pigmente, die gewählt werden aus der Gruppe der amphiphilen Metalloxide, insbesondere aus der Gruppe Titandioxid, Zinkoxid, Siliciumdioxid bzw. Silicate (z. B. Talkum), wobei die Metalloxide sowohl einzeln als auch im Gemisch vorliegen können. Dabei ist es im wesentlichen unerheblich, in welcher der gegebenenfalls natürlich vorkommenden Modifikationen die verwendeten amphiphilen Metalloxide vorliegen.

Vorteilhaft ist es, den mittleren Partikeldurchmesser der zur Kombination mit Bomitriden verwendeten Pigmente zwischen 1 nm und 200 nm, besonders vorteilhaft zwischen 5 nm und 100 nm zu wählen.

Vorteilhaft im Sinne der vorliegenden Erfindung ist es, die erfindungsgemäßen amphiphilen Bomitridpartikel mit unbehandelten, nahezu reinen Pigmentpartikeln zu kombinieren, insbesondere mit solchen, welche auch als Farbstoff in der Lebensmittelindustrie und/oder als Absorber von UV-Strahlung in Sonnenschutzmitteln verwendet werden können. Vorteilhaft sind beispielsweise die bei der Firma Merck erhältlichen Zinkoxid-Pigmente sowie solche, die unter den Handelsbezeichnungen Zinkoxid neutral bei Haarmann & Reimer oder NanoX von der Harcros Chemical Group erhältlich sind.

Erfindungsgemäß vorteilhaft ist ferner die Kombination von Bomitridpartikeln mit anorganischen Pigmenten, die oberflächlich wasserabweisend behandelt ("gecoatet") sind, wobei gleichzeitig ein amphiphiler Charakter dieser Pigmente gebildet werden bzw. erhalten bleiben soll. Diese Oberflächenbehandlung kann darin bestehen, daß die Pigmente nach an sich bekannten Verfahren mit einer dünnen hydrophoben Schicht versehen werden.

Ein solches Verfahren, das im folgenden am Beispiel von Titandioxid beschrieben wird, besteht beispielsweise darin, daß die hydrophobe Oberflächenschicht nach einer Reaktion gemäß

$$n\ TiO_2 + m\ (RO)_3\ Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Besonders vorteilhafte Kombinationspartner sind $TiO_2$-Pigmente, beispielsweise die mit Aluminiumstearat beschichteten, unter der Handelsbezeichnung MT 100 T bei der Firma TAYCA erhältlichen.

Eine weitere vorteilhafte Beschichtung der Kombinationspartner besteht aus Dimethylpolysiloxan (auch: Dimethicone), einem Gemisch vollmethylierter, linearer Siloxanpolymere, die endständig mit Trimethylsiloxy-Einheiten blockiert sind. Besonders vorteilhaft im Sinne der vorliegenden Erfindung ist die Kombination von Bomitriden mit Zinkoxid-Pigmenten, die auf diese Weise beschichtet werden.

Vorteilhaft ist ferner, wenn die neben Bornitrid verwendeten anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel beschichtet sind, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhafte Kombinationspartner sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Vorteilhaft im Sinne der vorliegenden Erfindung ist ferner die Kombination von Bomitriden mit einer Mischung verschiedener anorganischer, amphiphiler Pigmenttypen sowohl innerhalb eines Kristalls, beispielsweise als Eisenmischoxid oder Talkum (Magnesiumsilicat), als auch durch Mischung mehrerer Metalloxidtypen innerhalb einer Zubereitung.

Besonders vorteilhafte Kombinationspartner sind Magnesiumsilicate, beispielsweise die unter der Handelsbezeichnung Talkum Micron bei der Firma Grolmann erhältlichen.

Die erfindungsgemäßen amphiphilen Bornitridpartikel können ferner vorteilhaft mit weiteren Pigmenten kombiniert werden, beispielsweise mit Titandioxidpigmenten, die mit Octytsilanol beschichtet sind, und/oder mit Siliciumdioxidpartikeln, die oberflächlich wasserabweisend behandelt sind. Zur Kombination geeignete Siliciumdioxidpartikel sind beispielsweise sphärische Polyalkylsilsesquioxan-Partikel wie sie in der Europäischen Offenlegungsschrift 0 686 391 erwähnt werden. Solche Polyalkylsilsesquioxan-Partikel sind beispielsweise unter den Handelsbezeichnungen Aerosil R972 und Aerosil 200V bei der Firma Degussa erhältlich.

Weiter vorteilhaft werden die amphiphilen Bornitridpartikel mit mikrofeinen Polymerpartikeln kombiniert, welche in der Zubereitung in Form von Feststoffen vorliegen. Günstige Kombinationspartner im Sinne der vorliegenden Erfindung sind beispielsweise Polycarbonate, Polyether, Polyethylen, Polypropylen, Polyvinylchlorid, Polystyrol, Polyamide, Polyacrylate und dergleichen mehr.

Erfindungsgemäß geeignete Kombinationspartner sind beispielsweise mikrofeine Polyamid-Partikel, insbesondere die unter der Handelsbezeichnung SP-500 bei der Firma TORAY erhältlichen. Ferner vorteilhaft sind Polyamid 6- (auch: Nylon 6) bzw. Polyamid 12- (auch: Nylon 12) Partikel. Polyamid 6 ist das aus ε-Aminocapronsäure (6-Aminohexansäure) oder ε-Caprolactam aufgebaute Polyamid [Poly(ε-caprolactam)], und Polyamid 12 ist ein Poly(ε-laurinlactam) aus ε-Laurinlactam. Vorteilhaft im Sinne der vorliegenden Erfindung sind beispielsweise Orgasol® 1002 (Polyamid 6) und Orgasol® 2002 (Polyamid 12) von der Firma ELF ATOCHEM.

Weitere vorteilhafte mikrofeine Polymerpartikel, welche sich zur Kombination mit den erfindungsgemäßen Bornitridpartikeln eignen, sind mikrofeine Polymethacrylate. Solche Partikel sind beispielsweise unter der Handelsbezeichnung POLYTRAP® bei der Firma DOW CHEMICAL erhältlich.

Es ist insbesondere vorteilhaft, wenngleich nicht zwingend, wenn die als Kombinationspartner verwendeten mikrofeinen Polymerpartikel oberflächlich beschichtet sind. Diese Oberflächenbehandlung kann darin bestehen, daß die Polymerpartikel nach an sich bekannten Verfahren mit einer dünnen hydrophilen Schicht versehen werden. Vorteilhafte Beschichtungen bestehen beispielsweise aus Titandioxid ($TiO_2$), Zirkoniumdioxid ($ZrO_2$) oder auch weiteren Polymeren, wie beispielsweise Polymethylmethacrylat. Besonders vorteilhafte mikrofeine Polymerpartikel im Sinne der vorliegenden Erfindung sind beispielsweise die nach dem in der US-Patentschrift 4,898,913 beschriebenen Verfahren zur hydrophilen Beschichtung hydrophober Polymerpartikel erhältlichen.

Der mittlere Partikeldurchmesser der als Kombinationspartner verwendeten mikrofeinen Polymerpartikel wird vorzugsweise kleiner als 100 µm, besonders vorteilhaft kleiner als 50 µm gewählt. Dabei ist es im wesentlichen unerheblich, in welcher Form (Plättchen, Stäbchen, Kügelchen etc.) die verwendeten Polymerpartikel vorliegen.

Die erfindungsgemäßen amphiphilen Bornitridpartikel werden ferner vorzugsweise mit amphiphilen modifizierten Polysacchariden kombiniert, welche keine verdickenden Eigenschaften zeigen.

Solche amphiphilen Polysaccharide sind beispielsweise durch Umsetzung von Stärke mit mono-, bi- oder polyfunktionellen Reagenzien bzw. Oxidations-Mitteln in weitgehend polymeranalog verlaufenden Reaktionen erhältlich.

Diese Reaktionen basieren im wesentlichen auf Umwandlungen der Hydroxy-Gruppen der Polyglucane durch Veretherung, Veresterung oder selektive Oxidation. Dabei entstehen z. B. sogenannte Stärkeether und Stärkeester der allgemeinen Strukturformel

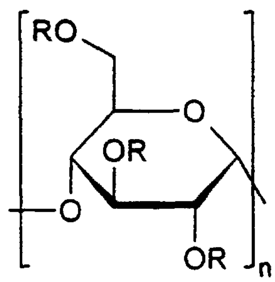

**Strukturformel (I)**

worin R beispielsweise ein Wasserstoff und/oder einen Alkyl- und/oder Aralkylrest (im Fall der Stärkeether) oder ein Wasserstoff und/oder einen organischen und/oder anorganischen Säure-Rest (im Fall der Stärkeester) darstellen kann. Stärkeether und Stärkeester sind vorteilhafte Kombinationspartner im Sinne der vorliegenden Erfindung.

Besonders vorteilhaft ist es, die erfindungsgemäßen Bomitridpartikel mit Stärkeethem zu kombinieren, z. B. mit solchen, die durch Veretherung von Stärke mit Tetramethylolacetylendiharnstoff erhältlich sind und welche als Amylum non mucilaginosum (nicht quellende Stärke) bezeichnet werden.

Insbesondere vorteilhaft ist ferner die Kombination von erfindungsgemäßen Bomitridpartikeln mit Stärkeestem und/oder deren Salzen, beispielsweise mit Natrium- und/oder Aluminiumsalzen niedrigsubstituierter Halbester der Stärke, insbesondere mit Natrium Stärke n-Octenylsuccinat der Strukturformel (I), worin R sich durch die folgende Struktur auszeichnet

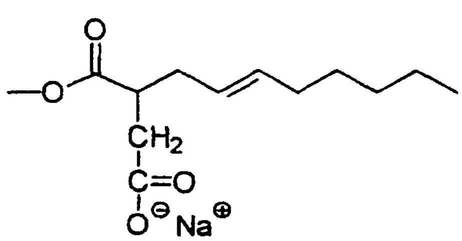

und welches z. B. unter der Handelsbezeichnung Amiogum® 23 bei der Firma CERESTAR erhältlich ist sowie mit Aluminium Stärke Octenylsuccinat, insbesondere mit den unter den Handelsbezeichnungen Dry Flo® Elite LL und Dry Flo® PC bei der Firma CERESTAR erhältlichen.

Vorteilhaft ist es, den mittleren Partikeldurchmesser der zur Kombination mit den erfindungsgemäßen amphiphilen Bomitridpartikel verwendeten, modifizierten Polysaccharide kleiner als 20 μm, besonders vorteilhaft kleiner als 15 μm zu wählen.

Die Liste der genannten modifizierten Polysaccharide, die mit den amphiphilen Bornitridpartikel kombiniert werden können, soll selbstverständlich nicht limitierend sein. Modifizierte Polysaccharide, die im Sinne der vorliegenden Erfindung vorteilhafte Kombinationspartner darstellen, sind auf zahlreichen, an sich bekannten Wegen, sowohl chemischer als auch physikalischer Natur erhältlich. Zur Herstellung solcher Polysaccharide sind auch neue Wege prinzipiell denkbar. Wesentlich dabei ist, daß die modifizierten Polysaccharide amphiphile Eigenschaften zeigen und daß sie nicht verdickend wirken.

Vorteilhaft in allen vorgenannten Fällen ist es, die Gesamtkonzentration aller Pigmente größer als 0,1 Gew.-%, besonders vorteilhaft zwischen 0,1 Gew.-% und 30 Gew.-% bezogen auf das Gesamtgewicht der Zubereitungen zu wählen, wobei die Konzentration an Bornitrid - ebenfalls bezogen auf das Gesamtgewicht der Zubereitungen - im Sinne der vorliegenden Erfindung vorzugsweise aus dem Bereich 0,1 Gew.-% bis 30 Gew.-%, vorteilhaft 0,5 Gew.-% bis 10 Gew.-% zu wählen ist.

Die erfindungsgemäßen Pickering-Emulsionen können als Grundlage für kosmetische oder dermatologische Formulierungen dienen. Diese können wie üblich zusammengesetzt sein und beispielsweise zur Behandlung und zur Pflege der Haut, als Lippenpflegeprodukt, als Deoprodukt und als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika üblichen Weise auf die Haut in ausreichender Menge aufgebracht.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Eine erstaunliche Eigenschaft der erfindungsgemäßen Zubereitungen ist, daß diese sehr gute Vehikel für kosmetische oder dermatologische Wirkstoffe in die Haut sind, wobei vorteilhafte Wirkstoffe Antioxidantien sind, welche die Haut vor oxidativer Beanspruchung schützen können.

Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden. Es ist dabei vorteilhaft, Antioxidantien als einzige Wirkstoffklasse zu verwenden, etwa dann, wenn eine kosmetische oder dermatologische Anwendung im Vordergrund steht, wie z. B. die Bekämpfung der oxidativen Beanspruchung der Haut. Es ist aber auch günstig, die erfindungsgemäßen Stiftzubereitungen mit einem Gehalt an einem oder mehreren Antioxidantien zu versehen, wenn die Zubereitungen einem anderen Zweck dienen sollen, z. B. als Desodorantien oder Sonnenschutzmittel.

Besonders vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropianat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Zink und dessen Derivate (z.B. ZnO, $ZnSO_4$), Selen und

dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den erfindungsgemäßen Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich-von 0,001 - 10 Gew.-%, bezogen auf das Gesamtgewicht der Formulierung, zu wählen.

Erfindungsgemäß können die Wirkstoffe (eine oder mehrere Verbindungen) auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin $B_1$, das Vitamin $B_{12}$ das Vitamin $D_1$, aber auch Bisabolol, ungesättigte Fettsäuren, namentlich die essentiellen Fettsäuren (oft auch Vitamin F genannt), insbesondere die gamma-Linolensäure, Ölsäure, Eicosapentaensäure, Docosahexaënsäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkemöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl, Eucerit® und Neocerit® .

Die Liste der genannten Wirkstoffe bzw. Wirkstoffkombinationen, die in den erfindungsgemäßen Pickering-Emulsionen verwendet werden können, soll selbstverständlich nicht limitierend sein.

Günstig sind auch kosmetische und dermatologische Zubereitungen, die in der Form eines Sonnenschutzmittels vorliegen. Vorzugsweise enthalten diese mindestens eine UV-A-Filtersubstanz und/oder mindestens eine UV-B-Fittersubstanz und/oder mindestens ein weiteres anorganisches Pigment aus der Gruppe der Oxide des Eisens, Zirkoniums, Siliciums, Mangans, Aluminiums, Cers und Mischungen davon sowie Abwandlungen, bei denen die Oxide die aktiven Agentien sind.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescremes gewöhnlich UV-A- bzw. UV-B-Filtersubstanzen eingearbeitet.

Vorteilhaft können erfindungsgemäße Zubereitungen Substanzen enthalten, die UV-Strahlung im UV-B-Bereich absorbieren, wobei die Gesamtmenge der Filtersubstanzen z. B. 0,1 Gew.-% bis 30 Gew.-%, vorzugsweise 0,5 bis 15 Gew.-%, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Enthalten die erfindungsgemäßen Emulsionen UV-B-Filtersubstanzen, können diese öllöslich oder wasserlöslich sein. Erfindungsgemäß vorteilhafte öllösliche UV-B-Filter sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- hinsichtlich der $C_3$-Achse des Triazingrundkörpers symmetrisch oder unsymmetrisch substituierte Triazinderivate, vorzugsweise 4,4',4"-(1,3,5-Triazin-2,4,6-triyltriimino)-tris-benzoesäure-tris(2-ethylhexylester) (symmetrisch) sowie 2,4-Bis-{[4-(2-Ethylhexyloxy)-2-hydroxy]-phenyl}-6-(4-methoxyphenyl)-1,3,5-triazin, 2,4-Bis-{[4-(3-sulfonato)-2-hydroxy-propyloxy)-2-hydroxy]-phenyl}-6-(4-methoxyphenyl)-1,3,5-triazin Natriumsalz, 2,4-Bis-{[4-(3-(2-Propy) oxy)-2-hydroxy-propyloxy)-2-hydroxy]-phenyl}-6-(4-methoxyphenyl)-1,3,5-triazin, 2,4-Bis-{[4-(2-ethyl-hexyloxy)-2-hydroxy]-phenyl}-6-[4-(2-methoxyethyl-carboxyl)-phenylamino]-1,3,5-triazin, 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, 2,4-Bis-{[4-(2-ethyl-hexyloxy)-2-hydroxy]-phenyl}-6-(1-methyl-pyrrol-2-yl)-1,3,5-triazin, 2,4-Bis-{[4-tris(trimethylsiloxy-silylpropyloxy)-2-hydroxy]-phenyl}-6-(4-methoxyphenyl)-1,3,5-triazin, 2,4-Bis-{[4-(2"-methylpropenyloxy)-2-hydroxy]-phenyl}-6-(4-methoxyphenyl)-1,3,5-triazin, 2,4-Bis-{[4-(1', 1', 1', 3', 5' ,5' ,5'-Heptamethylsiloxy-2-methyl-propyloxy)-2-hydroxy]-phenyl}-6-(4-methoxyphenyl)-1,3,5-triazin (unsymmetrisch),

- Benzotriazolderivate, vorzugsweise 2,2'-Methylen-bis-(6-(2H-benzotriazol-2-yl)-4-(1,1,3,3-tetramethylbutyl)-phenol)
- sowie an Polymere gebundene UV-Filter.

Vorteilhafte wasserlösliche UV-B-Filtersubstanzen sind z.B.:

- Salze der 2-Phenylbenzimidazol-5-sulfonsäure, wie ihr Natrium-, Kalium- oder ihr Triethanolammonium-Salz, sowie die Sulfonsäure selbst;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und deren Salze.

Die Liste der genannten UV-B-Filter, die in den erfindungsgemäßen Pickering-Emulsionen verwendet werden können, soll selbstverständlich nicht limitierend sein.

Es kann auch von Vorteil sein, in erfindungsgemäßen Pickering-Emulsionen UV-A-Filter zu verwenden, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion.

Weitere vorteilhafte UV-A-Filtersubstanzen sind die Phenylen-1,4-bis-(2-benzimidazyl)-3,3'-5,5'-tetrasulfonsäure:

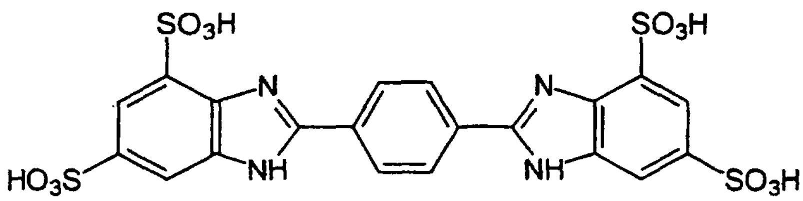

und ihre Salze, besonders die entsprechenden Natrium-, Kalium- oder Triethanolammonium-Salze, insbesondere das Phenylen-1,4-bis-(2-benzimidazyl)-3,3'-5,5'tetrasulfonsäure-bis-natriumsalz:

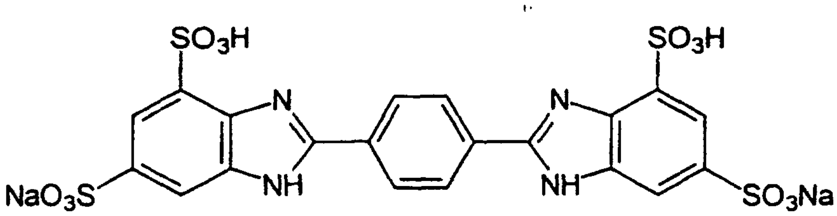

sowie das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-Sulfonsäure) bezeichnet wird und sich durch die folgende Struktur auszeichnet:

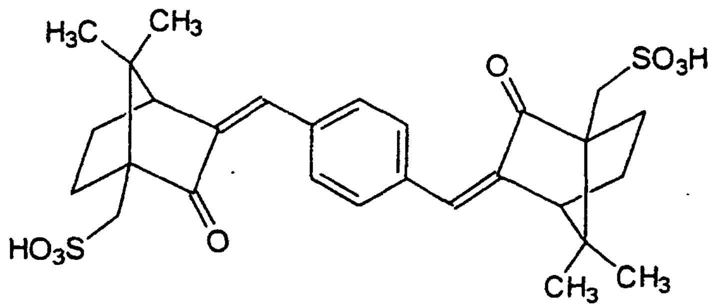

Vorteilhafte UV-Filtersubstanzen sind ferner sogenannte Breitbandfilter, d.h. Filtersubstanzen, die sowohl UV-A- als auch UV-B-Strahlung absorbieren.

Ein vorteilhaft zu verwendender Breitbandfilter ist beispielsweise das Ethylhexyl-2-cyano-3,3-diphenylacrylat (Octocrylen), welches von BASF unter der Bezeichnung Uvinul® N 539 erhältlich ist und sich durch folgende Struktur auszeichnet:

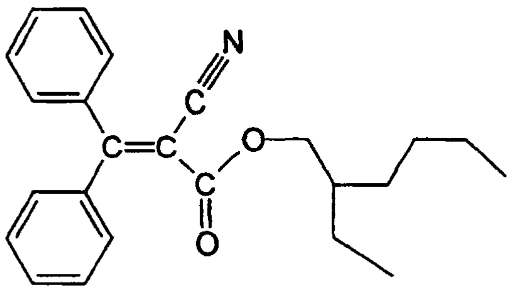

Auch Zubereitungen, die UV-A-Filter bzw. sogenannte Breitbandfilter enthalten, sind Gegenstand der Erfindung. Es können die für die UV-B-Kombination verwendeten Mengen eingesetzt werden.

Vorteilhaft können erfindungsgemäße Zubereitungen außerdem als Grundlage für kosmetische Desodorantien bzw. Antitranspirantien eingesetzt werden, so daß die vorliegende Erfindung in einer besonderen Ausführungsform Pickering-Emulsionen als Grundlage für kosmetische Desodorantien betrifft.

Kosmetische Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Bildung des Schweißes reduziert werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich.

Alle für Desodorantien bzw. Antitranspirantien gängigen Wirkstoffe können vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, llit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen W/O-Emulsionsstifte eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatriën-1-ol) sowie die in den Patentoffenlegungsschriften DE-37 40 186, DE-39 38 140, DE-42 04 321, DE-42 29 707, DE-43 09 372, DE-44 11 664, DE-195 41 967, DE-195 43 695, DE-195 43 696, DE-195 47 160, DE-196 02 108, DE-196 02 110, DE-196 02 111, DE-196 31 003,

DE-196 31 004 und DE-196 34 019 und den Patentschriften DE-42 29 737, DE-42 37 081, DE-43 24 219, DE-44 29 467, DE-44 23 410 und DE-195 16 705 beschriebenen Wirkstoffe bzw. Wirkstoffkombinationen. Auch Natriumhydrogencarbonat ist vorteilhaft zu verwenden.

Die Liste der genannten Wirkstoffe bzw. Wirkstoffkombinationen, die in den erfindungsgemäßen Pickering-Emulsionen verwendet werden können, soll selbstverständlich nicht limitierend sein.

Die erfindungsgemäßen kosmetischen Desodorantien können in Form von aus normalen Behältern auftragbaren wasserhaltigen, kosmetischen Zubereitungen vorliegen.

Die Menge der Antitranspiranswirkstoffe oder Desodorantien (eine oder mehrere Verbindungen) in den Zubereitungen beträgt vorzugsweise 0,01 bis 30 Gew.-%, besonders bevorzugt 0,1 - 20 Gew.-%, insbesondere 1 - 10 Gew.-%, bezogen auf das Gesamtgewicht der Zubereitung.

Die erfindungsgemäßen Stifte sind ferner hervorragende Vehikel für dermatologische Wirkstoffe. Insbesondere eignen sie sich als Träger für gegen Akne wirksame Substanzen. Akne ist eine Hauterkrankung mit verschiedenen Formen und Ursachen, gekennzeichnet durch nicht entzündliche und entzündliche Knötchen, ausgehend von verstopften Haarfollikeln (Komedonen), die zur Pustel-, Abszeß- und Narbenbildung führen kann. Am häufigsten ist die Acne vulgaris, die vorwiegend in der Pubertät auftritt. Ursächliche Bedingungen für die Acne vulgaris sind die Verhornung und Verstopfung der Haarfollikel-Mündung, die vom Blutspiegel der männlichen Sexualhormone abhängige Talgproduktion und die Produktion freier Fettsäuren und gewebeschädigender Enzyme durch Bakterien *(Propionibacterium acnes)*.

Daher ist es vorteilhaft, den erfindungsgemäßen Zubereitungen gegen Akne wirksame Substanzen zuzugeben, die beispielsweise gegen *Propionibacterium acnes* wirksam sind (etwa solche, die in DE-OS 42 29 707, DE-OS 43 05 069, DE-OS 43 07 976, DE-OS 43 37 711, DE-OS 43 29 379 beschrieben werden), aber auch andere gegen Akne wirksame Substanzen, beispielsweise all-trans-Retinsäure, 13-cis-Retinsäure und verwandte Stoffe) oder antientzündliche Wirkstoffe, beispielsweise Batylalkohol ($\alpha$-Octadecylglycerylether), Selachylalkohol ($\alpha$-9-Octadecenylglycerylether), Chimylalkohol ($\alpha$-Hexadecylglycerylether) und/oder Bisabolol sowie Antibiotika und/oder Keratolytika.

Keratolytika sind Stoffe, die verhornte Haut (wie z. B. Warzen, Hühneraugen, Schwielen und dergleichen mehr) erweichen, damit sich diese leichter entfernen läßt oder damit sie abfällt bzw. sich auflöst.

Alle gängigen gegen Akne wirksamen Substanzen können vorteilhaft genutzt werden, insbesondere Benzoylperoxid, Bituminosulfonate (Ammonium-, Natrium- und CalciumSalze von Schieferöl-Sulfonsäuren), Salicylsäure (2-Hydroxybenzoesäure), Miconazol (1-[2-(2,4-Dichlorbenzyloxy)-2-(2,4-dichlorphenyl)-ethyl)-imidazol) und Derivate, Adapalen (6-[3-(1-Adamantyl)-4-methoxyphenyl]-2-naphthoesäure), Azelainsäure (Nonandisäure), Mesulfen (2,7-Dimethylthianthren, $C_{14}H_{12}S_2$) sowie Aluminiumoxid, Zinkoxid und/oder feinverteilter Schwefel.

Die Menge der Antiaknemittel (eine oder mehrere Verbindungen) in den Zubereitungen beträgt vorzugsweise 0,01 bis 30 Gew.-%, besonders bevorzugt 0,1 - 20 Gew.-%, insbesondere 1 - 10 Gew.-%, bezogen auf das Gesamtgewicht der Zubereitung.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

**Beispiele:**

| | 1 | 2 | 3 | 4 | 5 | 6 |
|---|---|---|---|---|---|---|
| | W/O | W/O | W/O | O/W | O/W | O/W |
| Titandioxid (Eusolex T2000) | | 1 | 6 | | | 2 |
| Zinkoxid | | - | 4 | | | 4 |
| Silica (Aerosil R972) | | | 0,5 | | | |
| Talkum (Talkum Micron) | | 2 | | | | |
| Bornitrid | 5 | 3 | 2 | 5 | 3 | 5 |
| Natrium Maisstärke n-Octenylsuccinat | | | | | 1 | 1 |
| Orgasol® 1002 (Polyamid 6) | | 1 | | | 1 | |
| Caprylic/Capric Triglycerid | 5 | 5 | 5 | 20 | 20 | 20 |
| Octyldodecanol | 10 | | 5 | 20 | | 15 |

(fortgesetzt)

| | 1 | 2 | 3 | 4 | 5 | 6 |
|---|---|---|---|---|---|---|
| | W/O | W/O | W/O | O/W | O/W | O/W |
| Mineralöl | 10 | | 5 | 20 | | 20 |
| Butylenglykol Caprylat/Caprat | | 10 | 10 | | 20 | 7 |
| $C_{12-15}$ Alkylbenzoat | 10 | 10 | 10 | 5 | 20 | |
| Methylbenzyliden Campher | | 3 | - | | 4 | |
| Octyltriazon | | 1 | | | 4 | |
| Dibenzoylmethan | | 2 | | | 2 | |
| Konservierung | 0,5 | 0,5 | 0,5 | 0,5 | 0,5 | 0,5 |
| Glycerin | 5 | 10 | 3 | 5 | 5 | 5 |
| Phenylbenzimidazol Sulfonsäure | | 1 | | | 2 | |
| Carbomer | | | | 0,1 | | |
| NaOH 45 %ige Lösung in Wasser | | 0,3 | | 0,1 | 0,7 | |
| EDTA-Lösung | | 1 | | | 1 | |
| Wasser | ad 100 | ad 100 | ad 100 | ad 100 | ad 100 | ad 100 |

## Patentansprüche

1. Kosmetische oder dermatologische Zubereitungen, die feindisperse Systeme vom Typ Öl-in-Wasser oder Wasser-in-Öl darstellen, enthaltend

   1. eine Ölphase,
   2. eine Wasserphase,
   3. Bornitrid, welches sowohl hydrophile als auch lipophile Eigenschaften zeigt, welches also amphiphilen Charakter besitzt und sowohl in Wasser als auch in Öl dispergierbar ist und
   4. höchstens 0,5 Gew. % eines oder mehrerer Emulgatoren.

2. Zubereitungen nach Anspruch 1, **dadurch gekennzeichnet, daß** sie emulgatorfrei sind.

3. Zubereitungen nach einem der vorhergehenden Ansprüche, **dadurch gekennzeichnet, daß** weitere kosmetische oder pharmazeutische Hilfs-, Zusatz- und/oder Wirkstoffe enthalten sind.

4. Zubereitungen nach einem der vorhergehenden Ansprüche, **dadurch gekennzeichnet, daß** der Gehalt an Bomitrid zwischen 0,1 Gew.-% und 30 Gew.-% ist, bezogen auf das Gesamtgewicht der Zubereitungen.

5. Zubereitungen nach einem der vorhergehenden Ansprüche, **dadurch gekennzeichnet, daß** der mittlere Partikeldurchmesser der verwendeten Bornitridpartikel kleiner als 20 µm, besonders vorteilhaft kleiner als 15 µm ist.

6. Zubereitungen nach einem der vorhergehenden Ansprüche, **dadurch gekennzeichnet, daß** neben Bomitrid weitere Pigmente enthalten sind, insbesondere modifizierte Polysaccharide und/oder mikrofeine Polymerpartikel und/oder mikronisierte, anorganische Pigmente, die gewählt werden aus der Gruppe der amphiphilen Metalloxide, insbesondere aus der Gruppe Titandioxid, Zinkoxid, Eisenoxide bzw. Eisenmischoxide, Siliciumdioxid bzw. Silicate, wobei die Pigmente sowohl einzeln als auch im Gemisch vorliegen können.

7. Zubereitungen nach einem der vorhergehenden Ansprüche, enthaltend einen oder mehrere Zusatz- oder Wirkstoffe, gewählt aus der Gruppe der Antioxidantien und/oder UV-Schutzmittel.

8. Zubereitungen nach einem der vorhergehenden Ansprüche, enthaltend einen oder mehrere Zusatz- oder Wirkstoffe, gewählt aus der Gruppe der Adstringentien und/oder antimikrobiell wirksamen Stoffe und/oder gegen Akne wirksamen Stoffe.

## Claims

**1.** Cosmetic or dermatological preparations, which are finely dispersed systems of the oil-in-water or water-in-oil type, comprising

1. an oil phase,
2. an aqueous phase,
3. boron nitride which has both hydrophilic and lipophilic properties, i.e. has amphiphilic character, and is dispersible both in water and in oil, and
4. at most 0.5% by weight of one or more emulsifiers.

**2.** Preparations according to Claim 1, **characterized in that** they are emulsifier-free.

**3.** Preparations according to one of the preceding claims, **characterized in that** further cosmetic or pharmaceutical auxiliaries, additives and/or active ingredients are present.

**4.** Preparations according to one of the preceding claims, **characterized in that** the content of boron nitride is between 0.1% by weight and 30% by weight, based on the total weight of the preparations.

**5.** Preparations according to one of the preceding claims, **characterized in that** the average particle diameter of the boron nitride particles used is less than 20 µm, particularly advantageously less than 15 µm.

**6.** Preparations according to one of the preceding claims, **characterized in that**, in addition to boron nitride, further pigments are present, in particular modified polysaccharides and/or microfine polymer particles and/or micronized, inorganic pigments which are chosen from the group of amphiphilic metal oxides, in particular from the group consisting of titanium dioxide, zinc oxide, iron oxides or iron mixed oxides, silicon dioxide or silicates, where the pigments can be present either individually or as a mixture.

**7.** Preparations according to one of the preceding claims, comprising one or more additives or active ingredients selected from the group consisting of antioxidants and/or UV protectants.

**8.** Preparations according to one of the preceding claims, comprising one or more additives or active ingredients selected from the group consisting of astringents and/or antimicrobially effective substances and/or substances effective against acne.

## Revendications

**1.** Compositions cosmétiques ou dermatologiques formant des systèmes finement dispersés du type huile-dans-eau ou eau-dans-huile, contenant

1. une phase huileuse,
2. une phase aqueuse,
3. un nitrure de bore qui présente des propriétés hydrophiles ainsi que lipophiles, qui présente donc un caractère amphiphile et qui est dispersible dans l'eau ainsi que dans l'huile et
4. au plus 0,5% en poids d'un ou de plusieurs émulsifiants.

**2.** Compositions selon la revendication 1, **caractérisées en ce qu'**elles sont exemptes d'émulsifiant.

**3.** Compositions selon l'une quelconque des revendications précédentes, **caractérisées en ce qu'**elles contiennent d'autres adjuvants, additifs et/ou substances actives cosmétiques ou pharmaceutiques.

**4.** Compositions selon l'une quelconque des revendications précédentes, **caractérisées en ce que** la teneur en nitrure de bore est comprise entre 0,1% en poids et 30% en poids par rapport au poids total des compositions.

**5.** Compositions selon l'une quelconque des revendications précédentes, **caractérisées en ce que** le diamètre moyen des particules de nitrure de bore utilisées est inférieur à 20 µm, de manière particulièrement avantageuse inférieur à 15 µm.

**6.** Compositions selon l'une quelconque des revendications précédentes, **caractérisées en ce qu'**elles contiennent, en plus du nitrure de bore, d'autres pigments, en particulier des polysaccharides modifiés et/ou des particules polymères de la taille de l'ordre du micron et/ou des pigments inorganiques micronisés qui sont choisis dans le groupe des oxydes métalliques amphiphiles, en particulier dans le groupe constitué par le dioxyde de titane, l'oxyde de zinc, les oxydes de fer ou les oxydes mixtes de fer, le dioxyde de silicium ou les silicates, les pigments pouvant être présents seuls ou en mélange.

**7.** Composition selon l'une quelconque des revendications précédentes, contenant un ou plusieurs additifs ou substances actives choisis dans le groupe des antioxydants et/ou des agents de protection contre les UV.

**8.** Compositions selon l'une quelconque des revendications précédentes, contenant un ou plusieurs additifs ou substances actives, choisis dans le groupe des substances astreignantes et/ou des substances à activité antimicrobienne et/ou des substances efficaces contre l'acné.